# DEMANDE DE BREVET EUROPEEN

(11) **EP 2 502 616 A1**
(43) Date de publication de la demande: **26.09.2012**
(21) Numéro de dépôt: 11159067.5
(22) Date de dépôt: 21.03.2011
(51) Int. Cl.: A61K 9/00, A61K 9/12, A61K 31/00

(54) **Composition pharmaceutique nasale**

(71) Demandeur: Krayenbuhl, Matthew, 1009 Pully (CH); Kenyon, Michael, 1066 Epalinges (CH); Dos Santos, Antonio, 1092 Belmont-sur-Lausanne (CH)
(72) Inventeur: Krayenbuhl, Matthew, 1009, Pully (CH)
(74) Mandataire: Grosfillier, Philippe

(57) **Abrégé**

L'invention concerne une composition pharmaceutique de dose unitaire comprenant un corticoïde topique et une quinolone ou de l'acide fucidique, caractérisée par le fait que le rapport entre le poids du corticoïde topique et le poids de la quinolone ou de l'acide fucidique se situe entre 0.02 et 20 et la quantité de quinolone ou d'acide fucidique est inférieure à 1 mg. Cette composition peut être administrée par spray nasal et est utilisée pour le traitement des voies respiratoires supérieures et inférieures, en particulier des sinusites et des polypes nasaux.

## Description

### Domaine de l'invention

La présente invention concerne des compositions de dose unitaire comprenant un corticoïde topique et un antibiotique choisi dans la classe des quinolones ou un antibiotique tel que l'acide fucidique.

Ces compositions peuvent être avantageusement utilisées - mais pas exclusivement - dans le traitement de sinusites.

### Etat de la technique

Des compositions telles que décrites précédemment sont divulguées notamment dans la demande de brevet internationale WO 03/020219, la demande de brevet européen EP 1 894 559 A1 et le brevet américain US 7,691,094 B2.

### Exposé général de l'invention

Le problème que la présente invention se propose de résoudre réside dans une amélioration des traitements actuellement proposés, en particulier des traitements de la sinusite. Par amélioration, on entend « efficacité du traitement augmentée et/ou diminution de la toxicité et/ou minimisation des quantités utilisées (p.ex. d'antibiotique) ».

Dans l'invention, la solution du problème précité consiste en une composition pharmaceutique de dose unitaire dans laquelle le rapport corticoïde/antibiotique est particulièrement élevé en comparaison de celui que l'on observe dans les compositions de l'état de la technique.

Plus précisément, l'invention concerne une composition pharmaceutique de dose unitaire comprenant au moins un corticoïde topique et une quinolone ou l'acide fucidique, caractérisée par le fait que le rapport entre le poids du corticoïde topique et le poids de la quinolone ou de l'acide fucidique se situe entre 0.02 et 20 et la quantité de quinolone ou d'acide fucidique est inférieure à 1 mg. Plus particulièrement, la quantité de quinolone ou d'acide fucidique est comprise entre 10 et 400 µg.

La composition pharmaceutique de dose unitaire selon l'invention comprend un premier ingrédient actif sélectionné parmi le groupe des corticoïdes. Ces corticoïdes (également appelés stéroïdes, corticostéroïdes, glucocorticoïdes ou analogues de la cortisone) sont des composés généralement utilisés par voie locale (nasale, cutanée, ophtalmique,...). Des exemples de tels corticoïdes topiques incluent : le furoate de mométasone, le budésonide, la béclométasone le furoate de fluticasone, le propionate de fluticasone, le triamcinolone.

Le corticoïde préféré est le furoate de fluticasone et le triamcinolone.

La composition pharmaceutique de dose unitaire selon l'invention comprend en outre un second ingrédient actif sélectionné parmi le groupe des quinolones. Les quinolones forment une classe d'antibiotiques. Des exemples de telles quinolones incluent la norfloxacine, l'ofloxacine, la ciprofloxacine, la loméfloxacine, la lexofloxacine.

La quinolone préférée est la ciprofloxacine.

Le second ingrédient actif peut également être l'acide fucidique.

Dans un mode préféré de l'invention, la composition pharmaceutique de dose unitaire selon l'invention comprend du furoate de fluticasone et de la ciprofloxacine.

Dans un autre mode préféré de l'invention, la composition pharmaceutique de dose unitaire selon l'invention comprend de la budénoside et de la ciprofloxacine. Dans un autre mode préféré de l'invention, la composition pharmaceutique de la dose unitaire selon l'invention comprend le triamcinolone et la ciprofloxacine.

La composition pharmaceutique de dose unitaire selon l'invention peut également comprendre un ou plusieurs excipients acceptables sur le plan pharmaceutique. Ces excipients peuvent être choisis parmi les agents dispersants, solubilisants, stabilisants, conservateurs, antioxydants, etc. A titre d'exemple, la composition pharmaceutique de dose unitaire selon l'invention peut comprendre du mannitol et/ou du chlorure de benzalkonium. Elle peut comprendre également du sorbate de potassium, du glucose anhydre, de la cellulose dispersible, de la polysorbate 80, de l'édétate disodique.

La composition pharmaceutique de dose unitaire selon l'invention est plus particulièrement destinée au traitement des voies respiratoires supérieures et inférieures. Elle est pulvérisable et adaptée au conditionnement sous forme de spray nasal.

Les voies respiratoires supérieures (ou voies aériennes supérieures) sont extra thoraciques et comprennent le nez et les fosses nasales, la bouche, le pharynx (carrefour aérodigestif) et le larynx (gorge).

Les voies respiratoires inférieures (ou voies aériennes inférieures) sont intra-thoraciques et comprennent la zone de conduction et transition (voies extra-pulmonaires (trachée), voies intra-pulmonaires (bronches souches, bronches lobaires, bronchioles) et la zone respiratoire (conduits et sacs alvéolaires, alvéoles pulmonaires).

La composition pharmaceutique de dose unitaire selon l'invention est avantageusement formulée comme un liquide (solution, suspension, émulsion, etc.) en dose unitaire ou dans un flacon multi-doses pour une administration par spray dans la cavité nasale et les sinus pour le traitement des voies respiratoires. Cette composition inclut une poudre qui peut être mixée avec un diluant pour produire un liquide.

La composition de dose unitaire selon l'invention est utilisée dans le traitement des voies respiratoires supérieures, notamment dans le traitement de la sinusite, en particulier la sinusite chronique, et des polypes nasaux.

La composition pharmaceutique de dose unitaire selon l'invention est utilisée dans le traitement des voies respiratoires supérieures, notamment dans le traitement de la mucoviscidose.

La composition de dose unitaire selon l'invention dont le rapport entre le poids du corticoïde topique et le poids de la quinolone ou l'acide fucidique se situe entre 0.02 et 8 et la quantité de quinolone ou d'acide fucidique est inférieure à 1 mg est plus particulièrement utilisée pour le traitement des voies respiratoires inférieures.

L'invention a également comme objet une préparation pharmaceutique contenant plusieurs doses unitaires selon l'invention et se présentant sous forme de spray nasal.

Un autre objet de l'invention concerne une dose de composition pharmaceutique selon l'invention destinée à un usage d'une journée et pour une narine comprenant 150 à 50 µg de corticoïde topique et de 150 à 200 µg de quinolone.

Un autre objet de l'invention concerne l'utilisation de la composition de dose unitaire selon l'invention pour le traitement des voies respiratoires, ladite composition devant être administrée sous forme de spray deux fois, de manière consécutive, dans chaque narine et ce, une ou deux fois par jour.

### Exposé détaillé de l'invention

L'invention est décrite plus en détail ci-après au moyen d'exemples :

Une composition pharmaceutique de dose unitaire comprenant du budénoside et de la ciprofloxacine dans un rapport de 0.25 a été administrée par spray nasal, chaque dose unitaire ou pulvérisation nasale délivrant 25 µg de budénoside et 100 µg de ciprofloxacine. La composition comprend en outre au moins du mannitol et du chlorure de benzalkonium comme excipients. Une dose de deux pulvérisations par narine et par jour a été prescrite à chaque patient. Une pulvérisation nasale correspond à environ 100 µL de liquide.

Une composition pharmaceutique de dose unitaire comprenant du furoate de fluticasone et de la ciprofloxacine dans un rapport de 0.4 a également été administrée par spray nasal, chaque dose unitaire ou pulvérisation nasale délivrant 30 µg de furoate de fluticasone et 75 µg de ciprofloxacine. La composition comprend en outre au moins du mannitol et du chlorure de benzalkonium comme excipients. Une dose de deux pulvérisations par narine et par jour a été prescrite à chaque patient.

Une composition pharmaceutique de dose unitaire comprenant du triamcinolone et de la ciprofloxacine dans un rapport de 0,5 à également été administrée par pulvérisation nasale délivrant 37,5 µg de triamcinolone et 75 µg de ciprofloxacine.

Environ 1000 patients souffrants d'une sinusite chronique ou de polypes nasaux ont été testés avec un traitement journalier durant 4 à 8 semaines. Un effet positif a été observé et une amélioration notoire a été observée.

Il va sans dire que l'invention n'est pas limitée aux exemples illustrés ci-dessus.

## Revendications

1. Composition pharmaceutique de dose unitaire comprenant au moins un corticoïde topique et une quinolone ou de l'acide fucidique, **caractérisée par le fait que** le rapport entre le poids du corticoïde topique et le poids de la quinolone ou de l'acide fucidique se situe entre 0.02 et 20 et la quantité de quinolone ou d'acide fucidique est inférieure à 1 mg.

2. Composition pharmaceutique de dose unitaire selon la revendication 1, dans laquelle le rapport entre le poids du corticoïde topique et le poids de la quinolone est de 0.25 et la quantité de quinolone est de 100 µg.

3. Composition pharmaceutique de dose unitaire selon la revendication 1, dans laquelle le rapport entre le poids du corticoïde topique et le poids de la quinolone est de 1 et la quantité de quinolone est de 75 µg.

4. Composition pharmaceutique de dose unitaire selon l'une des revendications précédentes, dans laquelle le corticoïde topique est sélectionné dans le groupe de molécules suivant : le furoate de mométasone, le budésonide, la béclométasone, le furoate de fluticasone, le propionate de fluticasone, la triamcinolone.

5. Composition pharmaceutique de dose unitaire selon l'une des revendications précédentes, dans laquelle la quinolone est sélectionnée dans le groupe de molécules suivant : l'ofloxacine, la norfloxacine, la ciprofloxacine, la loméfloxacine, la lexofloxacine.

6. Composition pharmaceutique de dose unitaire selon la revendication 4 ou 5 dans laquelle le corticoïde est le furoate de fluticasone et la quinolone est la ciprofloxacine.

7. Composition pharmaceutique de dose unitaire selon la revendication 4 ou 5 dans laquelle le corticoïde est la triamcinolone et la quinolone est la ciprofloxacine.

8. Composition pharmaceutique de dose unitaire selon l'une des revendications précédentes comprenant en outre un excipient acceptable sur le plan pharmaceutique tel que le mannitol et/ou le chlorure de benzalkonium.

9. Composition pharmaceutique de dose unitaire selon l'une des revendications précédentes dans laquelle le rapport entre le poids du corticoïde topique et le poids de la quinolone est compris entre 0.25 et 1, la quantité de quinolone est comprise entre 75 µg et 100 µg, le corticoïde topique est le furoate de fluticasone et la quinolone est la ciprofloxacine, les excipients sont au moins le mannitol et le chlorure de benzalkonium.

10. Composition pharmaceutique de dose unitaire selon l'une des revendications 1 à 8, dans laquelle le rapport entre le poids du corticoïde topique et le poids de la quinolone est compris entre 0.25 et 1, la quantité de quinolone est comprise entre 75 µg et 100 µg, le corticoïde topique est le triamcinolone et la quinolone est la ciprofloxacine, les excipients sont au moins le mannitol et le chlorure de benzalkonium.

11. Composition pharmaceutique de dose unitaire selon l'une des revendications précédentes pour son utilisation dans le traitement des voies respiratoires supérieures.

12. Composition pharmaceutique de dose unitaire selon la revendication 11 pour son utilisation dans le traitement de la sinusite, en particulier de la sinusite chronique.

13. Composition pharmaceutique de dose unitaire selon l'une des revendications 1 à 10 pour son utilisation dans le traitement des voies respiratoires inférieures.

14. Composition pharmaceutique de dose unitaire selon la revendication 13 pour son utilisation dans le traitement de la mucoviscidose.

15. Préparation pharmaceutique contenant plusieurs doses unitaires selon l'une des revendications précédentes, **caractérisée par le fait qu'**elle se présente sous forme de spray nasal.

16. Dose de composition pharmaceutique selon l'une des revendications précédentes destinée à un usage d'une journée et pour une narine comprenant de 150 à 50 µg de corticoïde topique et de 150 à 200 µg de quinolone.

17. Utilisation de la composition pharmaceutique de dose unitaire selon l'une des revendications 1 à 14 pour le traitement des voies respiratoires, **caractérisée en ce que** ladite composition doit être administrée sous forme de spray deux fois dans chaque narine à raison d'une ou deux fois par jour.
